# EUROPÄISCHE PATENTSCHRIFT

(11) **EP 0 607 593 B1**
(45) Veröffentlichungstag und Bekanntmachung des Hinweises auf die Patenterteilung: **06.09.2000**
(21) Anmeldenummer: 93120536.3
(22) Anmeldetag: 20.12.1993
(51) Int. Cl.: A61K 35/14, C12N 5/00, C12P 21/00

(54) **Verfahren zur Gewinnnung von Zellkulturen mit erhöhtem Gehalt an körpereigenen Zytokinen**
Process for obtaining cell cultures with higher content of autologous cytokines
Procédé d'ontention de cultures cellulaires avec un taux amélioré de cytokines autogènes

(30) Priorität: 29.12.1992 DE 4244437
(43) Veröffentlichungstag der Anmeldung: 27.07.1994
(73) Patentinhaber: Kief, Horst, Dr. med., D-67069 Ludwigshafen (DE)
(72) Erfinder: Kief, Horst, Dr. med., D-67069 Ludwigshafen (DE)
(74) Vertreter: von Füner, Alexander, Prof.h.c. Dr.

(56) Entgegenhaltungen:
- EP-A- 0 265 548
- LYMPHOKINE AND CYTOKINE RESEARCH, Bd.10, Nr.5, Oktober 1991, NEW YORK Seiten 409 - 412 PAULESU, L. ET AL 'Studies on the biological effects of ozone: Induction of Tumor Necrosis Factor (TNF-alpha) on human leucocytes'
- HAEMATOLOGICA, Bd.75, Nr.6, 1990, ROMA Seiten 510 - 515 BOCCI, V. ET AL 'Studies on the biological effects of ozone 1. induction of interferon-gamma on human leucocytes'
- ENCYCLOPEDIA OF IMMUNOLOGY, 1992, ACADEMIC PRESS, IVAN M. ROITT, SEITEN 847-852

## Beschreibung

Zytokine sind Botenstoffe, die Signale zwischen immunkompetenten Zellen übertragen und die Empfängerzelle entweder zur Übernahme bestimmter Funktionen veranlassen und/oder zur Teilung anregen. Das vermehrte Wachstum bestimmter immunkompetenter Zelltypen im Blut darf als die wesentlichste Aufgabe der Zytokine betrachtet werden. Es gibt heutzutage nur einen Weg, Zytokine zu einem vertretbaren Marktpreis in größeren Mengen herzustellen: die Gentechnologie. Trotz inzwischen ausgereifter Technologie und Massenproduktion ist jedoch das Verfahren so aufwendig, daß die Zytokine derzeit noch zu den teuersten Medikamenten überhaupt auf dem Markt gehören. Obwohl in ihrer Struktur bekannt und im wesentlichen rein darstellbar, ist die Anwendung der Zytokine doch noch mit erheblichen Nebenwirkungen behaftet. Die Verabreichung gentechnologisch gewonnener Zytokine zeigt außerdem, daß bei aller Reinheit der Darstellung offenbar Fremdreaktionen doch nicht zu vermeiden sind, wie die teilweise starke Nebenwirkungsrate einschlägiger Präparate auf dem Markt belegt. Es haftet ihnen darüberhinaus ein prinzipieller Nachteil an: Da sie meist einen Zelltyp oder eine Gruppe als Zielzellen haben und das übrige Zusammenspiel des Immunsystems unberücksichtigt lassen, ist die Wirksamkeit gentechnologisch gewonnener und einzel verabreichter Zytokine unphysiologisch.

Das erfindungsgemäße Verfahren bietet demgegenüber die Möglichkeit, körpereigene Zytokine zu gewinnen, die - aufgrund ihrer Herkunft - dem eigenen Organismus erneut einverleibt, wesentlich besser verträglich sind und da sie im körpereigenen Medium belassen werden, auch nicht den oben geschilderten Nachteil der unphysiologischen Wirkung aufweisen.

Die Ozonbegasung von Vollblut oder Lymphozytekulturen und die dadurch bedingte Induktion von Tumornecrosisfaktor und Interferon wird in Lymphokine und Cytokine Research, 10(5), 1991, 409-412 beschrieben.

Die Möglichkeit immunmodulatorische Substanzen zu gewinnen, wurde erstmals in EP-B1-0 265 548 aufgezeigt. Die EP-B1-0 265 548 betrifft ein Verfahren zur Herstellung von keimabgetöteten oder in ihrer Virulenz abgeschwächten Substanzen (Suspensionen) auf extrakorporalem Wege, wobei die aus einem lebenden, erkranktem Individuum entnommenen Ursprungesubstanzen einer Oxidation insbesondere mittels eines OzonSauerstoffgemischs unterworfen werden und fraktioniert werden. Als Ursprungssubstanz kann Blut, Gewebe oder Urin eingesetzt werden. Gemäß EP-B1-0 265 548 kann die Ursprungssubstanz ferner auf mechanischem, enzymatischem oder osmotischem Wege auf Unterzellgröße zerkleinert, d.h. lysiert, werden. Darüberhinaus kann gemäß EP-B1-0 265 548 die Oxidation mehrfach durchgeführt werden und die behandelten Fraktionen wieder zu einer einheitlichen Dilution zusammengefügt werden. Praktisch geht man gemäß EP-B1-0 265 548 so vor, daß aus einem mit einem Oxidans behandelten Eigenblut die Erythrozyten abzentrifugiert werden, das Plasma abgezogen, nochmals getrennt mit einem Oxidans behandelt, die Erythrozyten nach (mehrmaligem) Waschen erneut mit einem Oxidans behandelt, teilweise in Aqua destillata suspendiert und so auf osmotischem Wege zum Platzen gebracht und nochmals mit einem Oxidans behandelt werden. In besonders gelagerten Fällen wird entweder dem Serum oder der Erythrozytensuspension mit einem Oxidans behandeltes Urinfiltrat zugefügt.

Gemäß EP-B1-0 265 548 werden also nach Desaggregation, durch Ozonolyse und Proteolyse aus einzelnen Blutfraktionen, aber auch aus Urin Lysate gewonnen, die bereits in diesem Patent als immunmodulatorische Substanzen beschrieben werden und deren klinische Wirksamkeit bei Autoimmunerkrankungen zwischenzeitlich unbestritten ist. Die Gewinnung von Gammainterferon aus Leukozytenkulturen, die mit Ozon begast wurden, ist zwischenzeitlich (1990) von Bocci nachgewiesen worden. Bocci konnte auch zeigen, daß die Angaben von Kief bezüglich der Konzentration des O₂/O₃-Gemisches eine optimale Ausbeute von Gammainterferon aus Vollblutkulturen gewährleistete. Wie man aus dieser Arbeit ersehen kann, beträgt die maximale Ausbeute bei diesem Vorgehen etwa 140 Picogramm pro ml. Nimmt man Vollblut oder andere Fraktionen, beispielsweise Leukozyten u. Lymphozyten zusammen für eine Kurzzeitkultur, lassen sich gemäß EP-B1-0 265 548 auch andere Zytokine gewinnen, beispielsweise Interleukin 2, das im Überstend (die Schicht der über den Zellen, die auf den Boden des Kulturgefäßes herabsinken, befindlichen Nährlösung) einer derartigen Kultur, verarbeitet nach diesem Verfahren, 40-fach über den Ausgangswerten liegt. Bei dem erfindungsgemäßen Verfahren können jedoch sowohl wesentlich höhere Ausbeuten erhalten werden als auch unterschiedliche Zytokingemische gewonnen werden.

Gegenstand der Erfindung ist ein Verfahren zur Gewinnung von Zellkulturen mit erhöhtem Gehalt an körpereigenen Zytokinen, bei dem Eigenblutkulturen eines Patienten mit einem Ozon-Sauerstoff-Gemisch behandelt und fraktioniert werden, und die erhaltenen Zellkulturen unter Zugabe von Lysaten, die durch Ozonolyse und Proteolyse von Blut oder Blutfraktionen tionen und/oder Urin gewonnen worden sind, als Stimulantien in an sich bekannter Weise gezüchtet werden.

Das erfindungsgemäße Verfahren geht also von der Anwendung des Verfahrens gemäß EP-B1-0 265 548 aus. Sein Vorzug besteht darin, die gemäß EP-B1-0 265 548 gewonnenen Lysate zur Mitoseanregung bei Leukozyten- und Lymphozytenkulturen, die gemäß EP-B1-0 265 548 vorbehandelt wurden, einzusetzen. Nach diesem Verfahren kann, je nach Auswahl der aus Serum, Leukozyten, Lymphozyten, Erythrozyten und/oder Urin gewonnenen Lysate, ein unterschiedliches Spektrum an Zytokinen gewonnen werden.

Der prinzipielle Ablauf des Verfahrens zur Gewinnung körpereigener Zytokine ist folgender:
Zunächst wird Vollblut ozonisiert und bebrütet. Danach werden Leukozyten durch Gewinnung des Buffycoats oder Lymphozyten über Trennmittel und Zonenzentrifugation erhalten. Diese Populationen haben bereits eine Selektion durch die Anreicherung von Sauerstoffradikalen im Vollblut erfahren, da radikalempfindliche Zellen unter dieser Behandlung zugrunde gehen und nur die resistenten Zellen überleben. Diese überlebenden Zellen werden sodann in üblicher Weise kultiviert und als Stimulantien Lysate hinzugegeben. Diese Lysate können beispielsweise nur aus Erythrozyten, Granulozyten oder Lymphozyten oder auch aus Serum des Patienten gemäß EP-B1-0 265 548 gewonnen werden.

Ein wesentlicher Vorteil des erfindungsgemäßen Verfahrens ist die ausschließliche Verwendung körpereigenen Materials. Dies wird sehr deutlich beim Vergleich mit den üblichen Nährlösungen, die bei Flüssigzellkulturen Verwendung finden und die praktisch immer Kälberserum enthalten. Das erfindungsgemäße Verfahren schließt eine Sensibilisierung gegen Tiereiweiß oder Übertragung von Zoonosen von vorneherein aus.

Je nach Wahl des Ausgangsmaterials lassen sich unterschiedliche Zytokingemische anregen: Bei Verwendung eines aus Serum gemäß EP-B1-0 265 548 gewonnenen Lysats beispielsweise ein besonderer Reichtum an Gammainterferon und Interleukin 2. Die Zellkulturen werden hernach in der bereits in EP-B1-0 265 548 beschriebenen Weise lysiert und als injizierbare Lösung gemäß arzneimittelrechtlichen Vorschriften aufgearbeitet. Derartig gewonnene Lysate regen im Organismus selbst in Verdünnungen von 1:1 000 000 und in Mengen von 0,1 ml subcutan verabreicht noch eine körpereigene Produktion von Gammainterferon an, die in einem ersten Screening 7-fach über den Ausgangswerten lagen. Interleukin 2-Spiegel wurden im Durchschnitt 6-fach über der Norm gemessen. Im Gegensatz dazu können die löslichen IL-2-Rezeptoren beim erstgeschilderten Verfahren um das zwei- bis dreifache reduziert werden, ein wesentliches Kriterium bei der Behandlung von Autoimmunerkrankungen.

Eine weitere Steigerung ist dadurch möglich, daß derartig angeregtes Blut erneut in üblicher Weise kultiviert und mit gemäß EP-B1-0 265 548 gewonnenen Lysaten beaufschlagt wird, sodaß man von einer Kulturkaskade mit noch höherem Zytokinspiegel sprechen kann. Der Einsatz, der nach EP-B1-0 265 548 gewonnenen Lysate als Stimulans für körpereigene Blutkulturen führt jedoch nicht nur zum Erhalt spezifischer Zytokine je nach Auswahl von Lysat und Kulturzelle, sondern auch zum vermehrten Ansprossen von Makrophagen und Killerzellen, die für den besonderen Einsatz bei malignen Erkrankungen geeignet sind. Die Begasung der Blutkulturen hat darüberhinaus den Vorteil, daß maligne Zellen aus entsprechendem Spenderblut durch Ozon in ihrer Mitose selektiv gehemmt (s. Sweet F., Ming-Shian Kao, Song-Chian D. Lee, Science Vol. 209,93-2), bei entsprechender Dosis und Gesamtmasse sogar selektiv vernichtet werden. Der besondere Aufwand, der bei der Aufbereitung von Eigenblutstammzellkulturen bei cytostatisch behandelten Krebspatienten erforderlich ist, wird bei Begasung mit Ozon weitgehend überflüssig.

Urin enthält neben Alpha 2 Glykoprotein, Transferrin, Hämosiderin, Immunglobulin A und Immunglobulin G. Außerdem lassen sich in unterschiedlichem Ausmaß Ketten oder Kettenbruchstücke von Immunglobulinen nachweisen, neben Ceruloplasmin, Haptoglobulinfragmenten sowie Spuren von Alpha 2 Makroglobulin. Diese Bestandteile sind Ausdruck einer sowohl passiven als auch aktiven Filtrationsleistung der Niere. Damit bietet Urin oder auch ein Proteingemisch, das aus Urin nach dem Stand der Technik konzentriert wurde, eine Stimulationsmöglichkeit für derartige Kulturen, wie sie durch Laborverfahren aus dem Blut kaum selektiert werden können. Deutlich wird das am Gehalt von Zytokinen, die man im Urin sowohl spontan als auch bei Nierenerkrankungen nachweisen kann (s. Sweet F., Ming-Shian Kao, Song-Chian D. Lee, Science Vol. 209,932).

Wird Urin gemäß EP-B1-0 265 548 aufbereitet und setzt es einer derartig vorbereiteten Kultur als Antigen zu, kann die Ausbeute an den Interleukinen 3, 4, 5 und 6 wesentlich erhöht werden bei einer zahlenmäßig deutlich erhöhten Anzucht an Eosinophilen.

Es ist leicht einsehbar, daß derartig gewonnene körpereigene Zytokine wesentlich billiger herzustellen sind, bei dem eingangs bereits beschriebenen Vorzug, den körpereigene Substanzen aufgrund ihrer Verträglichkeit grundsätzlich aufweisen. Dabei ist es nicht einmal notwendig, die Zytokine rein darzustellen, da gerade das Zusammenspiel von Zytokingemischen, körpereigenen Proteinen des Kulturmediums, Zellnukleotiden und Plasmaanteil offenbar einen stärkeren immunmodulatorischen Effekt ausüben, als das Zytokin für sich allein.

## Patentansprüche

1. Verfahren zur Gewinnung von Zellkulturen mit erhöhtem Gehalt an körpereigenen Zytokinen, bei dem Eigenblutkulturen eines Patienten mit einem Ozon-Sauerstoff-Gemisch behandelt und fraktioniert werden, und die erhaltenen Zellkulturen unter Zugabe von Lysaten, die durch Ozonolyse und Proteolyse von Blut oder Blutfraktionen und/oder Urin gewonnen worden sind, als Stimulantien gezüchtet werden.

2. Verfahren nach Anspruch 1, dadurch **gekennzeichnet**, daß die Zellkulturen Leukozyten-, Lymphozyten- oder Mischkulturen sind.

## Claims

1. A process for the production of cell cultures with increased content of endogenous cytokines comprising treating a patient's blood cultures with an ozone-oxygen mixture and fractionation, wherein the cell cultures are cultivated and lysates are added as stimulants, the lysates having been obtained by ozonolysis and proteolysis from blood or blood fractions and/or urine.

2. A process according to claim 1, **characterized** in that the cell cultures are whole blood, leukocyte, lymphocyte, or mixed cultures.

## Revendications

1. Procédé pour l'obtention de cultures cellulaires possédant une teneur supérieure en cytokines autogènes, dans lequel on traite des cultures du propre sang d'un patient avec un mélange d'ozone-oxygène et on les fractionne, et on fait croître à titre de stimulants les cultures cellulaires obtenues en ajoutant des lysats obtenus par ozonolyse et par protéolyse du sang ou de fractions du sang et/ou d'urine.

2. Procédé selon la revendication 1, caractérisé en ce que les cultures cellulaires sont des cultures de leucocytes, des cultures de lymphocytes ou des cultures mixtes.
